# EUROPEAN PATENT APPLICATION

(11) **EP 1 323 826 A1**
(43) Date of publication of application: **02.07.2003**
(21) Application number: 01907318.8
(22) Date of filing: 16.02.2001
(51) Int. Cl.: C12N 15/57, C07K 14/745

(54) **A CELL LINE EXPRESSING MUTATED HUMAN TISSUE - TYPE PLASMINOGEN ACTIVATOR, THE CONSTRUCTING STRATEGY THEREOF AND METHOD OF PREPARING EXPRESSED PROTEIN**

(30) Priority: 04.09.2000 WO PCT/CN00/00260
(71) Applicant: Xia, Jiahui, Changsha, Hunan Province 410078 (CN)
(72) Inventor: Xia, Jiahui, Changsha, Hunan Province 410078 (CN)
(74) Representative: Grünecker, Kinkeldey, Stockmair & Schwanhäusser Anwaltssozietät
(86) International application number: PCT/CN01/00127
(87) International publication number: WO 02/022832

(57) **Abstract**

The invention involves a cell line which can express human mutated tissue-type plasminogen activator (TNK-TPA), and its preparation methods. The collection No. of cell line in this invention is CCTCC C200006. We firstly use a DNA fragment from the short arms of human D, G group chromosomes or its homologous to construct a recombinant human source gene vector-TNK-TPA (collection number is CCTC m200032); TNK-TPA gene is then transferred into the target site of nucleolus organizing region (NOR) of human D, G group chromosomes in host cell HT1080 by the recombinant and the cell line is attained after screening, which can be used for manufacturing protein.

## Description

### SUMMARY OF THE INVENTION

The invention involves a cell line specifically expressing mutated human tissue-type plasminogen activator (TNK-TPA), cell line's construction strategy and its application in manufacturing recombinant gene medicine of human tissue-type plasminogen activator.

### BACKGROUND OF THE INVENTION

Human tissue-type plasminogen activator(t-PA) can dissolve local thrombus in blood vessel and recanalize the blocked vessel by activating plasminogen. At present, American Genetech Company is the only one can develop recombinant human t-PA(rt-PA) at a large scale by means of gene project technology and has developed successfully rt-PA medicine. The first generation of rt-PA made in this company has become of choice treatment to thrombotic diseases, but it has some shortcomings. First, it's price is rather high with $1375 per vial on international market; second, its function has some weak points: 1)short half-life, just 3 to 5 minutes; 2)low specific affinity with fibrin of gore; 3) activity and efficacy should be further improved. Much more medicine dose should be given because of short half-life. Low specificity and activity give rise to two results: on one hand, there is still 10-20% patient's thrombus can't be completely dissolved by rt-PA, on the other hand, it can results in fibrinolysis over all the body and affects normal cruor mechanism so as to cause severe complications such as bleeding, especially in skull and digestive tract. In terms of the above reasons, some foreign companies have got down to develop the second generation of rt-PA since the -PA was used in the clinical. They modified the rt-PA gene in order to eliminate or weaken the above weak points. TNK-TPA is a kind of mutated form which is universally researched. For example, Keyt BA, Paoni NF. Refino rt CJ, et al proclaimed "a Faster-acting And More Potent Form of Tissue Plasminogen Activator". ((Proc Natl Acad Sci USA) 1997 Apr 26; 91(9):3670-4.). It was constructed on the basis of three sites of t-PA gene site-directed mutation. Two sites locate in Kringlel function domain, T103N and Q117N; one locates in Protease domain, KHRR296-299AAAA.T103N adds a glycosylation site which increases specificity of combination of TNK-tPA and fibrin and decreases its cleanup rate; Q117N destroys combination of Kl region and mannose and the action domain of KUPFFER cell in liver, and prolongs half-life; KHRR296-299AAAA destroys functional region of PAI-1 and prolongs its half-life, too. As for the development of TNK-TPA, it is universal to adopt ordinary eukaryotic gene expression vector. using CHO cell as host cell, TNK-TPA integrate randomly into the expression system of host cell genome. For example. Wu Benchuan, et al announced the above expression system and preparation method of expressed protein in the article "Purification and identification of recombinant tissue-type plasminogen activator" published in <Development of Biochemistry and Biophysics>( 1997,24 (1):71-75)

### DETAILED DESCRIPTION OF THE INVENTION

The inventor discovered two families carrying an extra bi-satellite microchromosome (BM) in these two families with 2 and 3 generations, the BM does no harm to human body. The families show normal phenotype. It is reported 17 similar families in the world, and nobody conceived of assembling human gene vector using the small chromosome elements. The applicant put forward the structure. He found out that the chromosomes come from short arms of human D, G group chromosomes 13,14,15,21,22 by FISH technique. The short arms of human D, G group are nucleolus organizing region abundant in ribosome DNA (rDNA). These regions exist different polymorphisms with different length in human, and the genes in these regions can be actively transcribed in the cell metaphase. So he inferred that specific DNA fragments can be used as leading sequence if they were isolated from BM, and the gene of interest can be transferred into nucleolus organizing region in short arms of human D, G group chromosomes, the gene should be relatively highly, stably and harmlessly expressed. The following example can prove this strongly.

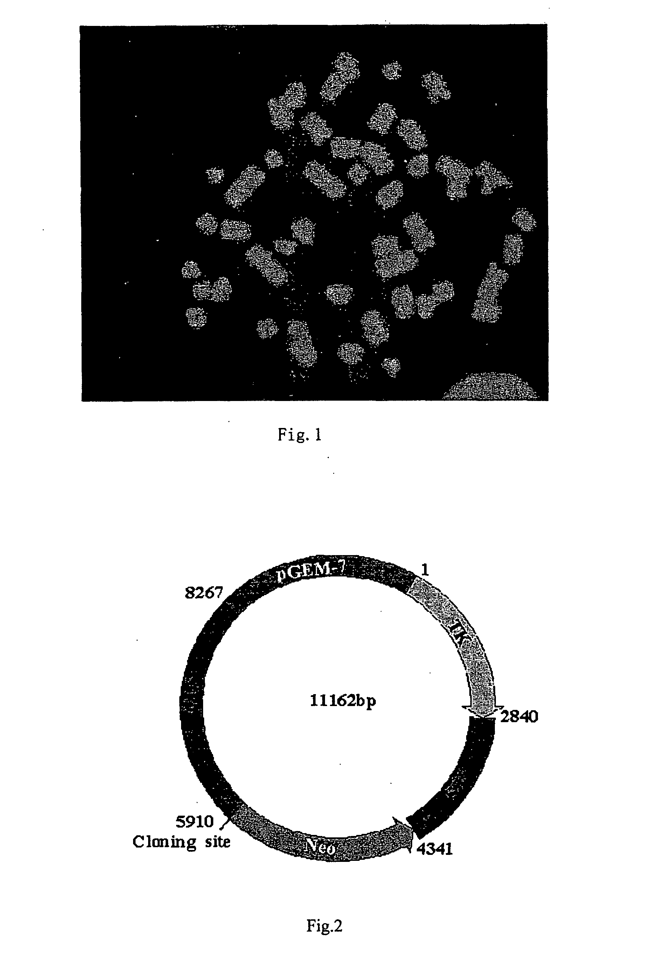

The inventor at first constructed specific pUC19 library of the microchromosomes by micro-dissection method and got single copy fragment by screening the library. The fragment proved by FISH technique comes from short arms of human D, G group chromosomes and the single copy was used as probe to screen human PAC genomic library. Then, a 120kb of BM specific DNA fragment which comes from BM and short arms of human D, G group chromosomes was confirmed by FISH technique (Fig. 1). Gene concerned important physiological function is not found by analyzing sequence of this BM specific fragment (BMSF). So it is safe to take it as target. The applicant further used a small fragment from BMSF as leading sequence to construct gene vector.

Based on above evidences, the applicant holds that a gene vector by using the DNA sequence without vital physiological function- related gene or a DNA sequence with identity to short arms of human D, G group chromosomes as the leading sequence can site-directly introduce target gene to the short arms of human D, G group chromosomes and the cell line for this invention can then be obtained.

Different vectors can be constructed on the basis of leading sequence according to the present technique, and then we can get recombinant vector-TNK-TPA by ligating TNK-TPA gene into the constructed vector. The construction methods of vector and recombinant vector-TNK-TPA are commonly used. Using the recombinant above, the target gene TNK-TPA can be also transferred into host cell by routine method.

The example of the invention gives in detail the identification of the 120kb specific DNA sequence from short arms of human D, G group chromosomes, the construction of the vector using a 3.8kb fragment from the 120kb BMSF as leading sequence. The full sequence of the vector is given in SEQ No 1. Then target gene is ligated into the vector and recombinant vector-TNK-TPA is achieved. The inserting site of TNK-TPA gene is at 5910 of the vector. The bacterial strain contained recombinant vector-TNK-TPA is preserved in the China Typical Culture Collection Center (Wuhan University, China. Wuhan 430072) on Sept. 29, 2000, collection number is CCTCCM200032. The preservator names it Escherichia coli JM 109/JH-S/pNS-TPA.

According to the way above, the invention got a novel cell line with target gene TNK-TPA integrated in short arms of D, G group chromosomes of HT1080 cell. The cell line was accepted by China Typical Culture Collection Center (Wuhan University, China. Wuhan 430072) on August 18, 2000, and preservation number is CCTCC C200006. The cell line is human fibrosarcoma cell line, which is human gene-transferred cell. The preservator names it human fibrosarcoma cell line /JH-2/TPA. The preserved is a pure cultivative cell line, the culture condition is 10% calf serum in DMEM , PH is 7.0-7.4 ,37°C.

The above cell line can substitute CHO transformed form to prepare TNK-TPA.

The purified protein expressed by the cell line of the invention has been confirmed to be the target protein by Western blotting and amino acid sequence analysis.

The invention obtained a novel cell line by transferring site-directly recombinant human-source gene vector/TNK-TPA cDNA into D,G group chromosomes of human HT1080 cell and gave a method to produce TNK-TPA. Compared with the existing technology, it dramatically decreases cost of TNK-TPA because of its efficient and stable expression.
Figure 1 shows FISH mapping of 120kb fragment cloned in PAC;
Figure 2 is a circular map of the gene vector described in the invention (length of gene vector sequence: 11162bp); pGEM-7 (8267-11162):vector replication component and prokaryote screening system; TK(1-2840): eukaryote negative screen gene, using TK promoter and TK polyA signal; Neo(4342―5910):eukaryote positive screen gene, using sv40 promoter and sv40 polyA signal; GLS (2841―4341,5911―8267):leading sequence for gene targeting; Cloning site (5910): insert-site of target gene;
Figure 3 is FISH mapping of targeted TNK-TPA gene in positively transformed cell colony, this revealed that TNK-TPA gene has been site-directly transferred into short arms of D,G group chromosomes by the vector;
Figure 4 is Western blotting result of purified TNK-TPA protein, 1∼4 are purified TNK-TPA protein, "―"suggests negative control;

### EXAMPLE ONE

### Preparation of gene leading sequence in the invention

1 Isolation of PAC clone with gene leading sequence
1.1 Construct BM specific pUC19 library through micro-dissection, PCR, microcloning (Deng H-X, Yoshirua K, Dirks RW, et al. Hum Genet 1992,89:13.)
1.2 Identification of BM specific single copy DNA
(1)preparation of colony matrix membrane: draw a 14×14 square wells on two nylon membranes, labeled as A and B, and put into two plates with solid LB, respectively, then pick white clone randomly from library plates and inoculate to the 14×12 square wells of two membranes with same coordinate, add 100ng single copy DNA to the 13th line as positive control, and then add 100ng gDNA to 14th line as negative control, two plates are incubated for 10∼12 hours at 37°C,and membrane B is stored at 4 °C, remove membrane A from plate, and treat on a filter paper soaked by following solution: 10%SDS, 5 minutes,0.5N NaOH/1.5M NaCl, 3 minutes, 1.5M NaCl/0.5M Tris.HCl, 3 minutes, 2×SSC/0.2M Tris.HCl, 10 minutes, 80 °C vacuum dry 2 hours, keep for use.
(2)Preparation of gDNA probe
Sample 50∼75ng gDNA, add asepsis water to 11ml, boil and denature at 100 °C for 10 minutes, the following system is used as random primer labeling reaction:

| 2mM dNTP(dATP⁻) | 3µl |
|---|---|
| primer mixture | 2µl |
| Klenow enzyme | 1µl |
| α-³²p-dATP | 3µl |

vortex and mix up thoroughly, incubate 30 minutes at 37 °C. Add 8µl stop mixture, pass through G-50 column to purify probe, withdraw 1/10 of the mixture to conduct liquid scintillation.
(3)Hybridization: colony matrix membrane is immersed in 2 × SSC 10 minutes, wipe out lightly colony fragments on membrane surface, 65°C, pre-hybridize in 5ml hybridization solution over 30 minutes. Sample probe solution to boil and denature 10minutes at 100 °C according to the concentration of 1.2×10⁶cpm/ml and measure liquid scintillation value, add 5ml fresh hybridization solution , hybridize with colony lattice membrane over 12 hours at 65°C,wash membrane according to following conditions: 2×SSC/0.1%SDS, 10 minutes at room temperature, 2× SSC/0.1%SDS, 10 minutes at 65°C,0.1%×SSC/0.1%SDS,10 minutes at 65°C, -70°C, autoradiograph, none or weak hybridization signal is primarily regarded as single copy.
(4)Sequence analysis, Southern blotting: pick clone without hybridization signal on the corresponding position of membrane B, culture it in small scale, sequence plasmid DNA extracted, the sequence of single copy DNA shows no similarity compared with the database of GenBank. At last, digest the plasmid by EcoR I enzyme and isolate the insert DNA by random primer method, label the insert by α-³²p-dATP and hybridize with gDNA digested by EcoRI on a piece of nylon membrane, those showing 1 or 2 hybridization bands are single copy DNA clones.
1.3 Identification of PAC clone with BM specific sequence and short arms of human D, G group chromosomes
(1) Screen human PAC gDNA and acquire positive clone
(2) Label 260bp single copy probe P8-7 with α-³²p-dATP by random primer method→purify probe by G-50 column (medium granularity) →ready for use 4°C →immerse 7 PAC membranes in 2×SSC 10 minutes→pre-hybridize 3 hours,55°C →denature probe 10 minutes, 100°C→add to 50ml hybridization solution purchased commercially to a final concentration of 4.6×10⁵cpm/ml and hybridize with PAC membrane 1 hour 65°C→wash membrane: 2×SSC ,once a time 10 minutes at room temperature, 2 × SSC/0.1%SDS,10 minutes at 65°C twice→apply to X-ray film, autoradiograph at 70°C 12 hours→develop X-ray film→read the positive clones number following instruction.
(2) Pick randomly positive clones and number from five different plates, purchase PAC clone.

1.4 Hybridization of the DNA fragment in PAC clone with metaphase cell confirm that the insert DNA in positive PAC clone originates from short arms of human D, G group chromosomes through FISH, as the figure 1 shows.
The above experimental methods refer to the *Molecular Cloning* written by J Sambrook et al., second Edition, Cold Spring Harbor laboratory Press,1989.
2. Isolation of the leading sequence.
Main material: β-agarase(Bio-Labs), Not I Agarose
(1) Digest the plasmid PAC169 by Not I enzyme
(2) Isolate an around 120kb insert DNA through PFGE.
   Pulse electrophoresis conditions: electrophoresis buffer: 0.5×TBE High strength Analytical Grade Agarose: (Bio-Rad, low Melting point agarose LMP)1%,
   Switch time: from two to fifteen second,
   electrophoresis time:18h.voltage: 6v/cm,angle:120° ,temperature: 14 °C.
(3) After electrophoresis, stain the gel with EB (0.2ug/ml) thirty minutes. According to the Marker display, cut that 120kb DNA band with sterile knife.
(4) Treat the gel cut out by β-agarase and precipitate with enthanol.

### EXAMPLE 2

### Preparation of gene vector in the invention:

1. The construction of gene vector and induction of target gene
1.1 Construct gene vector
   1.1.1 Nsi I and Stu I (blunt enzyme) digest PAC DNA, run the sample on a common agarose gel, recover the 3.8kb DNA and purify the DNA by electric elution.
   1.1.2 pGEM-TK vector DNA is digested with Hind III, and then digested with Klenow further to generate the blunt ends.
   1.1.3 The blunt ends of pGEM-TK/Hind III is further digested with Nsi I.
   1.1.4 The purified 3.8kb/Nsi I+Stu I digested DNA and the Nsi I digested pGEM-TK are ligated for 17 hours at 16°C.
   1.1.5 The ligated product is transformed into JM109 competent bacteria, culture 18 hours at 37°C in an ampicillin plate.
   1.1.6 Pick randomly monoclones and identify the positive clones with Nsi I and Nhe I double digestion.
   1.1.7 The plasmid pCDN-GPR is digested with Xba I and Nhe I to acquire the Xbal+Nhel digested Neo gene.
   1.1.8 Construct pNS2 gene vector by ligating the Xbal+Nhel digested Neo gene with the pGEM-TK-3.8kb/Nhe I fragment.
2.1 Induction of TNK-TPA gene
   2.1.1 Clone TNK-TPA (CDS) into pcDNA3.1(-).
   2.1.2 Design the primers TPCF and TPCR to amplify TNK-TPA gene and expression element (CMV promoter and BGH polyA signal), Avr II restrictive sites are added to the two ends of the primers. 
Primer Sequence: TPCF: ATGCATCCTAGGGGAGGTCGCTGAGTAGTG
AvrII
TPCR: TGCATGCCTAGGTACCCCCTAGAGCCCAG
AvrII
   2.1.3 Digest TNK-TPA gene and expression element (CMV promoter and BGH polyA signal) with AvrII, ligate it to the pNS2 vector digested with Nhe I.
   2.1.4 Transform the ligated product into recombinant JM109 E.coci and gain bacterial strain containing vector-TNK-TPA (collection number is CCTCC M200032).

   The above experimental methods refer to the *Molecular Cloning* written by .J Sambrook et al., second edition, Cold Spring Harbor laboratory Press.1989.

3 The gene vector extraction.
3.1 Material:
3.1.1 QIAGE Plasmid Maxi Kit
3.1.2 Culture medium: liquid LB

| Trypton | 5g |
|---|---|
| Yeast extract | 2.5g |
| NaCI | 2.5g |
| Add H₂O to 500ml | |
| Sterilize by autoclaving | |

3.1.3 Ampicillin: 100mg/ml(1000×)
3.2 Method:
1) Pick positive monoclones and inoculate them in 3ml LB (Amp+), incubate for 1 h at 37°C, 250rpm.
2) Inoculate 100ul of primary culture in 100 ml LB (Amp+), incubate for 16 h at 37 °C,250rpm.
3) Harvest the bacteria by centrifugation at 6000×g for 15 minutes at 4°C
4) Resuspend the bacterial pellet in 10ml of buffer P1.
5) Add 10ml of buffer P2, mix gently but thoroughly 6 times, and incubate at room temperature for 5 minutes.
6) Add 10ml of chilled buffer P3, mix gently by inverting 6 times, and incubate on ice for 20 minutes.
7) Centrifuge at 2000× g for 30 min at 4°C.
8) Transfer supernatant containing plasmid DNA promptly into a 40ml high-speed centrifuge tube and centrifuge at 2000× g for 15 min at 4°C.
9) Equilibrate a QIGEN tip 500 by applying 10ml buffer QBT.
10) Transfer the supernatant to the QIGAE-tip 500 and allow it to pass through this column.
11) Wash the Q1GAE-tip with 30ml buffer QC.
12) Elute DNA with 15ml QF and collect elution.
13) Precipitate DNA by adding 10.7ml (0.7 volumes) isopropylalcohol to the elution, mix up.
14) Centrifuge immediately at 15000×g for 30min at 4°C.
15) Remove the supernatant, wash DNA pellet with 5ml of 70% ethanol and centrifuge at 15000× g for 10 min.
16) Remove the 70% ethanol, air-dry the pellet for 10 min, and dissolve the DNA in TE solution.

### EXAMPLE 3

Transfer the recombinant vector-TNK-TPK acquired in the example 2 into host cell HT1080, obtain the required cell lines after screening, and express in vitro, confirms site-directed integration and high efficient expression.
1 Material:
1.1 Cell: HT1080.
   Culture medium: high sugar DMEM+10%FBS(HT1080) EMEM+10%FBS.
   1.2 Electroporation apparatus: purchased from Bio-Rad Company.

2.Methods
1) Culture the cells in a 75cm ² culture bottle to 70%∼80% confluence.
2) Harvest the cells and wash with HeBs Buffer for two times, then account the cells.
3) Centrifuge at 15000rpm at 4°C for 10 min.
4) Resuspend the cells with a reasonable volume of HeBS Buffer to make the cell density to 10⁶∼10⁷/ml.
5) Add 0.8 ml suspension (about 10µg DNA vector) to an 0.4cm electroporation cuvette
6) Electroporate the suspension, parameters: 260V, 550uF for 11∼13 ms.
7) Transfer the shocked cells to a 75cm² culture flask, add 14ml culture medium with ampicillin/streptomycin, then culture in 37°C, 5%CO₂ for 24∼48 hrs.
8) Screen the cell with G418 (final concentration of 300µg/ ml), change medium once every 2-3days, in parallel, normal cells is used as control.
9) Calculate the survival clone number in transfected cell after normal cells completely die in 7-10 days, and then add G418 (150µg/ml) to maintain it.
10) Continue to screen the transfected cells with GCV (the ultimate concentration of 500ng/ml)
11) Most cells die in 7-10 days, add GCV 250µg/ml to remain living cells or withdraw GCV, culture the cell up to 70%∼80% confluence, and determine its expressing activity of the transfected gene.

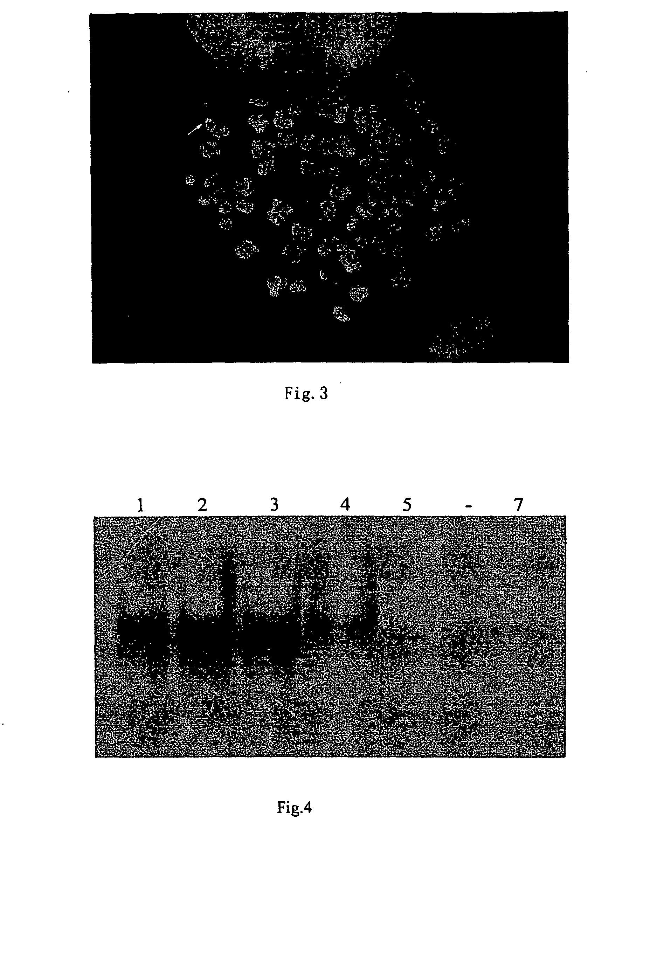

3.RESULTS
The TNK-TPA gene with the vector were transferred into HT1080 cells by electroporation and the positive cell lines were obtained after positive and negative screening (Collection NO. CCTCC: C200006), and confirmed to be site-directed insertion by FISH (Fig. 3) .The results of activity assay of TNK-TPA refer to Table 1.
Expression activities are: negative control HT1080 cells is 0 U/10⁶ cells/ 24 hrs. the positive HT1080 cells is 408 U/10⁶/24 hrs after transfer and 407 U/10⁶ cells/ 24 hrs after 95 days. The expression of TNK-TPA is of highly stability. In addition. expressed protein of TNK-TPA is proved by Western Blotting and amino acid sequencing (Fig. 4).

**Table 1:**

| The results of activity assay of TNK-TPA for positive HT1080 cells(ug/10⁶ cells/24hrs) | | | |
|---|---|---|---|
| The days of transfer | T1* | The days of transfer | T15* |
| 33 | 408 | 54 | 188 |
| 37 | 396 | 58 | 204 |
| 60 | 411 | 95 | 114 |
| 68 | 430 | | |
| 74 | 430 | | |
| 88 | 441 | | |
| 90 | 440.9 | | |
| 95 | 407 | | |

| | | | |
|---|---|---|---|
| *T1, T15 are two positive cell lines expressing TNK-TPA | | | |

4 The technology of TNK-TPA protein preparation
1)Culture TNK-TPA/ HT1080 cell line with DMEM medium till 80% confluence, and continue to culture them with serum-free, collect the supernatant after 24 hrs which containing expressed TNK-TPA;
2) Filtrate the supernatant with 0.22 µm filter membrane.
3) Concentrate the filtrate 7 times with 10KD ultrafiltration membrane.
4) Pass through PROSEP-LYSINEII chromatography media column.
5) Wash out the nonspecific protein absorbed on the column with 5× column volume of 0.05M PB buffer (pH 7.0).
6) Elute TNK-TPA protein bound to the column with 0.2M Arginine / 0.05M PB (PH7.5);
7) Desalt protein solution with 3KD ultrafiltration membrane, lyophilize it into powder dosage

Sequence Listing

<110> XIA, JIAHUI

<120> A Cell Line Expressing Mutated Human Tissue-Type Plasminogen
Activator, Its Construction Strategy and Method of Expressed
Protein Preparation

<150> PCT/CN00/00260

<151> 04 September 2000 (04.09.00)

<160> 1

<211> 11162bp

<212> DNA

<213> target gene vector

<400>

1 GGGCGAATTG GGCCCGACGT CGCATGCTCC TCTAGACTCG AGGAATTCTA
51 CCGGGTAGGG GAGGCGCTTT TCCCAAGGCA GTCTGGAGCA TGCGCTTAAG
101 CAGCCCCGCT GGGCACTTGG CGCTACACAA GTGGCCTCTG GCCTCGCACA
151 CATTCCACAT CCACCGGTAG GCGCCAACCG GCTCCGTTCT TTGGTGGCCC
201 CTTCGCGCCA CCTTCTACTC CTCCCCTAGT CAGGAAGTTC CCCCCCGCCC
251 CGCAGCTCGC GTCGTGCAGG ACGTGACAAA TGGAAGTAGC ACGTCTCACT
301 AGTCTCGTGC AGATGGACAG CACCGCTGAG CAATGGAAGC GGGTAGGCCT
351 TTGGGGCAGC GGCCAATAGC AGCTTTGCTC CTTCGCTTTC TGGGCTCAGA
401 GGCTGGGAAG GGGTGGGTCC GGGGGCGGGC TCAGGGGCGG GCTCAGGGGC
451 GGGGCGGGCG CCCGAAGGTC CTCCGGAGGC CCGGCATTCT GACGCTTCAA
501 AAGCGCACGT CTGCCGCGCT GTTCTCCTCT TCCTCATCTC CGGCCTTTCG
551 ACCTGCAGCG ACCCGCTTAA CAGCGTCAAC AGCGTGCCGC AGATCTTGGT
601 GGCGTGAAAC TCCCGCACCT CTTCGGCAAG CGCCTTGTAG AAGCGCGTAT
651 GGCTTCGTAC CCCTGCCATC AACACGCGTC TGCGTTCGAC CAGGCTGCGC
701 GTTCTCGCGG CCATAGCAAC CGACGTACGG CGTTGCGCCC TCGCCGGCAG
751 CAAGAAGCCA CGGAAGTCCG CCTGGAGCAG AAAATGCCCA CGCTACTGCG
801 GGTTTATATA GACGGTCCTC ACGGGATGGG GAAAACCACC ACCACGCAAC
851 TGCTGGTGGC CCTGGGTTCG CGCGACGATA TCGTCTACGT ACCCGAGCCC

901 GATGACTTAC TGGCAGGTGC TGGGGGCTTC CGAGACAATC GCGAACATCT
951 ACACCACACA ACACCGCCTC GACCAGGGTG AGATATCGGC CGGGGACGCG
1001 GCGGTGGTAA TGACAAGCGC CCAGATAACA ATGGGCATGC CTTATGCCGT
1051 GACCGACGCC GTTCTGGCTC CTCATATCGG GGGGGAGGCT GGGAGCTCAC
1101 ATGCCCCGCC CCCGGCCCTC ACCCTCATCT TCGACCGCCA TCCCATCGCC
1151 GCCCTCCTGT GCTACCCGGC CGCGCGATAC CTTATGGGCA GCATGACCCC
1201 CCAGGCCGTG CTGGCGTTCG TGGCCCTCAT CCCGCCGACC TTGCCCGGCA
1251 CAAACATCGT GTTGGGGGCC CTTCCGGAGG ACAGACACAT CGACCGCCTG
1301 GCCAAACGCC AGCGCCCCGG CGAGCGGCTT GACCTGGCTA TGCTGGCCGC
1351 GATTCGCCGC GTTTACGGGC TGCTTGCCAA TACGGTGCGG TATCTGCAGG
1401 GCGGCGGGTC GTGGCGGGAG GATTGGGGAC AGCTTTCGGG GACGGCCGTG
1451 CCCGCCCCAG GGTGCCGAGC CCCAGAGCAA CGCGGGCCCA CGACCCCATA
1501 TCGGGGACAC GTTATTTACC CTGTTTCGGG CCCCCGAGTT GCTGGCCCCC
1551 AACGGCGACC TGTACAACGT GTTTGCCTGG GCCTTGGACG TCTTGGCCAA
1601 ACGCCTCCGT CCCATGCACG TCTTTATCCT GGATTACGAC CAATCGCCCG
1651 CCGGCTGCCG GGACGCCCTG CTGCAACTTA CCTCCGGGAT GGTCCAGACC
1701 CACGTCACCA CCCCCGGCTC CATACCGACG ATCTGCGACC TGGCGCGCAC
1751 GTTTGCCCGG GAGATGGGGG AGGCTAACTG AAACACGGAA GGAGACAATA
1801 CCGGAAGGAA CCCGCGCTAT GACGGCAATA AAAAGACAGA ATAAAACGCA
1851 CGGGTGTTGG GTCGTTTGTT CATAAACGCG GGGTTCGGTC CCAGGGCTGG
1901 CACTCTGTCG ATACCCCACC GAGACCCCAT TGGGGCCAAT ACGCCCGCGT
1951 TTCTTCCTTT TCCCCACCCC ACCCCCCAAG TTCGGGTGAA GGCCCAGGGC
2001 TCGCAGCCAA CGTCGGGGCG GCAAGCCCTG CCATAGCCAC GGGCCCCGTG
2051 GGTTAGGGAC GGGGTCCCCC ATGGGGAATG GTTTATGGTT CGTGGGGGTT
2101 ATTATTTTGG GCGTTGCGTG GGGTCAGGTC CACGACTGGA CTGAGCAGAC
2151 AGACCCATGG TTTTTGGATG GCCTGGGCAT GGACCGCATG TACTGGCGCG
2201 ACACGAACAC CGGGCGTCTG TGGCTGCCAA ACACCCCCGA CCCCCAAAAA
2251 CCACCGCGCG GATTTCTGGC GCCGCCGGAC GAACTAAACC TGACTACGGC
2301 ATCTCTGCCC CTTCTTCGCT GGTACGAGGA GCGCTTTTGT TTTGTATTGG
2351 TCACCACGGC CGAGTTTCCG CGGGACCCCG GCCAGGACCT GCAGAAATTG
2401 ATGATCTATT AAACAATAAA GATGTCCACT AAAATGGAAG TTTTTCCTGT
2451 CATACTTTGT TAAGAAGGGT GAGAACAGAG TACCTACATT TTGAATGGAA
2501 GGATTGGAGC TACGGGGGTG GGGGTGGGGT GGGATTAGAT AAATGCCTGC
2551 TCTTTACTGA AGGCTCTTTA CTATTGCTTT ATGATAATGT TTCATAGTTG
2601 GATATCATAA TTTAAACAAG CAAAACCAAA TTAAGGGCCA GCTCATTCCT
2651 CCCACTCATG ATCTATAGAT CTATAGATCT CTCGTGGGAT CATTGTTTTT
2701 CTCTTGATTC CCACTTTGTG GTTCTAAGTA CTGTGGTTTC CAAATGTGTC
2751 AGTTTCATAG CCTGAAGAAC GAGATCAGCA GCCTCTGTTC CACATACACT
2801 TCATTCTCAG TATTGTTTTG CCAAGTTCTA ATTCCATCAG AAGCTCCTTA
2851 ATTTTATACC ACTGACTTAT TTTGAAGGCT GCTATAAGAA ACAGCCCTAT
2901 GAAACTGGTA TTTTCCTACT GCAAGGTGGC TACTTTAAGA CAATTTTTCA
2951 TTGCATTCTA TCAAGGGATG TCTTATTATT ATATCATTAT ATCAAGTGAT
3001 GTTATAAATA GTAAGAATCA GATTAAGGGC TCATATGTCC TTCTTTGTAT
3051 TGACTGTTGA AAAGGTATGG GGCCAAATTT GTAGTTTGTC TGGAATTACA

3101 TATTTTTGGG GGTCTCTATT ATCTTCATAC TTATCCTATC TAAATTTTCC
3151 ATTGCCAAAT TTCCTTACTT ATTTTTAGTT TTATCCTATT GCTCATGTAT
3201 TTTTATGTCT CCATAAGTCT ATTTTGGAAA AAGGCAGAGT ACTCATAATT
3251 TTAGTATATC TTTTAGCTTT ATGTTGCCAT AAACCTTTCA TTATATACAT
3301 GATCAACAAC AGCAAATTAT CTCACTTCAG TATTTAGTTT ATTATTTTAC
3351 AAACTGATTT ATGATTGCTA ACATGTAACT GAAGGTATAC ACTATTAGAA
3401 CACAGTTTTC AGTAGAAAGT AGCACTGCCA TTGAGTAAAA AAATGTTCTA
3451 ACATTAGAGC AACATTCTTA TACAAGTTTG CATGTTGTTT ACTGAGGTCT
3501 AAAGCATGAC TACACAAAAG GCTGAATAAA ATTCAGATTC TTACATACAC
3551 ATAAAATTGT TTTATTGAGA TGACAAAGTA TATTTATTAT GCCACCCAGA
3601 ATATAATCCA CTCTGATAAC TGCCAGTGTA TGCACTTGCT GAAGTAACTC
3651 AGTACATAAA TGGTAGCCAC AACAGTTGCT GTGCATGAAA GTTCTTCTCT
3701 TCCAGATTGA AGAGTGTACA ATCTAAAGCA TTTTAAAACT TTAAATCCCT
3751 TATTAGCTTA AATATAATTT AAAATTTTAG TTTGCCGTAC CTATAATTTG
3801 TCTGTACACT AGGTTACTAA GGGTGATATG ATTACATATG TGGATACAAA
3851 ATAATTTTAA TGGAAAATGA AATTAGGGTA CTCAACAAAG ATAAAGGGTA
3901 ATGATCATGT ACACTAACCG TATTTGAGAT TAGTTTAAGC CTGGGGTAGC
3951 TATACTTATG TTTCACAGAC CTTGAGAAGA TAGGGAAAAA AAGCTTTTAT
4001 CAACATTGCT AAGGAACAGG TAAAAGCTAA CATTAGGTAA CTAAGAGGTG
4051 ACATAAAAAA GACTGAATAA AATATCATGG AGGTTTCATA ATAAGATTGG
4101 AAATTCCATA GACTAGGAGA GAAAAGATCC CAAAATATAC ATGCTCATTG
4151 GGAAAACAGC TAGTAAGAAC AAGGAGAGAT CTCTATTTAA TGATACAATA
4201 GTAGAGTTAT AATTTCCTGT ATATTGTAAA TTTCAAGCAT TTAAACATTT
4251 TCATTGAATT ATAAAATATT ATTTGTAAAA GAAAGAAAAA CAGCACAACT
4301 GCAGATTACA GATGACTAAG ATAGATGAAT CATGAAAAGG TGCTAGATTG
4351 TGAGCGGATA ACAATTTCAC ACAGGAAACA GCTATGACCA TGATTACGCC
4401 AAGCTCTCGA CGGGATCGCG GCCGCGATCC AGACATGATA AGATACATTG
4451 ATGAGTTTGG ACAAACCACA ACTAGAATGC AGTGAAAAAA ATGCTTTATT
4501 TGTGAAATTT GTGATGCTAT TGCTTTATTT GTAACCATTA TAAGCTGCAA
4551 TAAACAAGTT GGGGTGGGCG AAGAACTCCA GCATGAGATC CCCGCGCTGG
4601 AGGATCATCC AGCCGGCGTC CCGGAAAACG ATTCCGAAGC CCAACCTTTC
4651 ATAGAAGGCG GCGGTGGAAT CGAAATCTCG TGATGGCAGG TTGGGCGTCG
4701 CTTGGTCGGT CATTTCGAAC CCCAGAGTCC CGCTCAGAAG AACTCGTCAA
4751 GAAGGCGATA GAAGGCGATG CGCTGCGAAT CGGGAGCGGC GATACCGTAA
4801 AGCACGAGGA AGCGGTCAGC CCATTCGCCG CCAAGCTCTT CAGCAATATC
4851 ACGGGTAGCC AACACTATGT CCTGATAGCG GTCCGCCACA CCCAGCCGGC
4901 CACAGTCGAT GAATCCAGAA AAGCGGCCAT TTTCCACCAT GATATTCGGC
4951 AAGCAGGCAT CGCCATGGGT CACGACGAGA TCCTCGCCGT CGGGCATGCT
5001 CGCCTTGAGC CTGGCGAACA GTTCGGCTGG CGCGAGCCCC TGATGCTCTT
5051 CGTCCAGATC ATCCTGATCG ACAAGACCGG CTTCCATCCG AGTACGTGCT
5101 CGCTCGATGC GATGTTTCGC TTGGTGGTCG AATGGGCAGG TAGCCGGATC
5151 AAGCGTATGC AGCCGCCGCA TTGCATCAGC CATGATGGAT ACTTTCTCGG
5201 CAGGAGCAAG GTGAGATGAC AGGAGATCCT GCCCCGGCAC TTCGCCCAAT
5251 AGCAGCCAGT CCCTTCCCGC TTCAGTGACA ACGTCGAGCA CAGCTGCGCA

5301 AGGAACGCCC GTCGTGGCCA GCCACGATAG CCGCGCTGCC TCGTCTTGCA
5351 GTTCATTCAG GGCACCGGAC AGGTCGGTCT TGACAAAAAG AACCGGGCGC
5401 CCCTGCGCTG ACAGCCGGAA CACGGCGGCA TCAGAGCAGC CGATTGTCTG
5451 TTGTGCCCAG TCATAGCCGA ATAGCCTCTC CACCCAAGCG GCCGGAGAAC
5501 CTGCGTGCAA TCCATCTTGT TCAACCATGG TGGATCGATC CAAGCTCCCA
5551 ACACAACTAT GTCAGAAGCA AATGTGAGGA GCAACTGATC CTACCTCACC
5601 TTATATGCTC TGCCCTGGCT CCTGCCCTCT CTATCCTGTG TGAGCAGATT
5651 GGCCCTTACC AAGGTGTGGC TCTACGGAAT CAGGCTTCGG TGATGACAAG
5701 CATATTTCTC CCTAGAATGC TGTGCCACTC ACTGGCTTAG GAGTCTCAGC
5751 TCTGGGTACT CCCTCTGAAT AATGTTTGTC CTTATCTGTG CAGAGAACAC
5801 TGTCTCTAAA GCATCCTTTT TGGCAACGCA TTTGCTCAAT CAACTACTGA
5851 ATTGGTGTTA AAATTAATTT TCCTTTTTTT CTCATTATGC AAATAAGAAA
5901 TTGAGAAGCA AAGCTAGCAG AGATTTCTAT CACACCTATC AGGGATACAC
5951 AATTTCCAAG AATTTCAGAA GTGTTTGGTG TTCCTATTAA CATAAATCCG
6001 GAAATAACAC CTGAGTGAAC TGTCTTCTAA TTCTTCAACT GGATGGCTTT
6051 TTAGTGTAAA AGATGTTGAA TACTGATTGA CTTTTTAATA ATTTTATAGT
6101 ATATGTCAGA AATATTGCAC AGTCCCTATT TACATCATTC TACAGTGGTT
6151 TTTAAAATGT TTTAAGAATA AAAAACATGA AAACTTTATT TGATTTTTCT
6201 GAGGAAATAA CTTTTTGGAT TTAATTTCAA TGAAACCGTT GATAACATTT
6251 CCCTCCCCAA CAATCTCTGG CAACGATCCC TCAGATTTTA ATGATTATGT
6301 ATTATTACCT TTTAATACAA GTAGAATAAC ACTCAGGGAA TTTACAACAT
6351 TTGTTATTTT CAGTAAATAC ATTGGTTGAA GTTTAAAAGT CTATCCGTAG
6401 TAAACTTACA TCTTTCAGGA GCTTGGTCAA TGTGTTCTGG ACAAAGCAGG
6451 AAGATGTGAC TGAAATCCTG AAAGGAGCCG GCTCCTGCAG CACAAGGATA
6501 ATGATACATC TGGGTACATT TCTCTTCACA GCATTTGATA GTGGCTCCAA
6551 AGTGCTTACA AAATGCACAT TGCTGAAAGG GGTAAAGGAG AGAAATCTCT
6601 TTATAAAACC TTGAAAAGGA ATATTTAAAT ATAAGCTGGG AAGGTATAAA
6651 AAACTCTCTG TAACATCACA AGTAAACAAA TTGAACCTGC AAAATATTAA
6701 ACAAAGGATT CATTAAAAAT AATAAAATCT ACATTACTCA ATTTAGTGCT
6751 TTGTGTGCTA CCAACTCATC CTTCCATTCA AATTAGAAAG TTAGAATTTC
6801 ATTCCTTATA TTTTCAAAAA TAAATTGTGA AGCATTTTAG AAACAAAACC
6851 TAAAATTTTT TTTTAAAAGC AAATAGTAAT ATGGTTAAAG GGGCAGGTTT
6901 CTATATTGAG GATTATTATA AAGTTTTTAA ATCCTACCAA AACTAGTAAT
6951 AGGAACATAT ATTATTTATG AGACATATTA CTATTTTTTA CCCTGCCTAA
7001 AAATAAATAC AAATAAATTC ATCAATTATA AGTTAACAGG GACACAAATG
7051 GTTAAAGACT CACACACAAA AAAAACAAAA CTACATACTT CAATGTAGCA
7101 ATCAACTTCA AATTTCTTAA CAAAAGATGG AAATGTTGGG GAAAAAATTA
7151 GTCATCTGGT ATCTTTCCCA TTTCAACCTG CCTCCATTAT CTTGCAAGTG
7201 GTAAAATGCA CAGAAATAAG CCTCAAACAA GAGGGGCAGT CTAGGGCAAG
7251 TGAACACATA AGTCGGAAGA AATTATGTAA AATGTTGCAT TTACTTATTC
7301 AGTTTTCCCT TAGAATGATT CACAAACTCT TCCTCATTCT CCCAAGTCCA
7351 TTTTGAGTAT CATTTTCTTT GAAGAGAGTC TGATGGGCCC TGTACTATAC
7401 AGTATGAAAT CTCTCTGTGG GAAATGACTA TCTAACATAA ATTTTTGTTT
7451 ACACCGTTAC ATGGTACCTA CTTGCTTATG CCATTACATG ATCAGTTTAC

7501 CTTTTTCTCA ACCTAATCCA AGATCCTTCA ATTGAGGCAC TATACTATCT
7551 TTGTATCCAA AGCACCAAAA ATGCTGCTTC AAACAGGCCC TAATAGATAG
7601 GTGTTCCTAT ACATATACCA AAAAGACTTA ACTTTTGGTG ATCTTGTTTG
7651 TGAGTGTGGC TCATAAACAG CTTAGTTGAG ATAACTGGAG CCTCATGTAG
7701 CAGAGACAGT TGGACCCTGC TAACATTACT GTGGATATCT TCACATGTTA
7751 CTACATTGAC TTTATATTCT GCTAATTAAC CAGGGACTAC AGTAGTTAAA
7801 ATTATAATTG TTTTCAATGT TTTATGTGTA AATCTGTATC TCACATACTA
7851 TCAAACTCTT CCTCACTGTC ATCAGTCTAC TGCATTGAAT CCAACATAAC
7901 AAAGCTAAAT GACTCCTGAG GGCTGAATCA GAAAGAAGAA AAGAAAGAGA
7951 TACAAAACTT TAGTCGGCCC GGTGGCTCAC ACCTGTAATC CCAGCACTTT
8001 GGAAGGCCAA GGCGGGCGGA TCACGAGGTC AGGAGATCGA GACCATCCTG
8051 GCTGATACAG TGAAACTCCA TCTCTACTGA AAATACAAAA AATTAGCTGG
8101 ACGTGGTGGT GGGCACCTGT AGTCCCAGCT ACTCAGGAGG CTGAAGCAGG
8151 AGAAGCTTCT AAATAACTCA TAAACACTAA TTACTGTTGT GACACTTTAA
8201 TTTTATACAA TATTTATAAG TATACAGAAT AACATTTCAG TGCTATTTTG
8251 GCACTCAAGG GTATTAATGC ATAGCTTGAG TATTCTATAG TGTCACCTAA
8301 ATAGCTTGGC GTAATCATGG TCATAGCTGT TTCCTGTGTG AAATTGTTAT
8351 CCGCTCACAA TTCCACACAA CATACGAGCC GGAAGCATAA AGTGTAAAGC
8401 CTGGGGTGCC TAATGAGTGA GCTAACTCAC ATTAATTGCG TTGCGCTCAC
8451 TGCCCGCTTT CCAGTCGGGA AACCTGTCGT GCCAGCTGCA TTAATGAATC
8501 GGCCAACGCG CGGGGAGAGG CGGTTTGCGT ATTGGGCGCT CTTCCGCTTC
8551 CTCGCTCACT GACTCGCTGC GCTCGGTCGT TCGGCTGCGG CGAGCGGTAT
8601 CAGCTCACTC AAAGGCGGTA ATACGGTTAT CCACAGAATC AGGGGATAAC
8651 GCAGGAAAGA ACATGTGAGC AAAAGGCCAG CAAAAGGCCA GGAACCGTAA
8701 AAAGGCCGCG TTGCTGGCGT TTTTCGATAG GCTCCGCCCC CCTGACGAGC
8751 ATCACAAAAA TCGACGCTCA AGTCAGAGGT GGCGAAACCC GACAGGACTA
8801 TAAAGATACC AGGCGTTTCC CCCTGGAAGC TCCCTCGTGC GCTCTCCTGT
8851 TCCGACCCTG CCGCTTACCG GATACCTGTC CGCCTTTCTC CCTTCGGGAA
8901 GCGTGGCGCT TTCTCATAGC TCACGCTGTA GGTATCTCAG TTCGGTGTAG
8951 GTCGTTCGCT CCAAGCTGGG CTGTGTGCAC GAACCCCCCG TTCAGCCCGA
9001 CCGCTGCGCC TTATCCGGTA ACTATCGTCT TGAGTCCAAC CCGGTAAGAC
9051 ACGACTTATC GCCACTGGCA GCAGCCACTG GTAACAGGAT TAGCAGAGCG
9101 AGGTATGTAG GCGGTGCTAC AGAGTTCTTG AAGTGGTGGC CTAACTACGG
9151 CTACACTAGA AGGACAGTAT TTGGTATCTG CGCTCTGCTG AAGCCAGTTA
9201 CCTTCGGAAA AAGAGTTGGT AGCTCTTGAT CCGGCAAACA AACCACCGCT
9251 GGTAGCGGTG GTTTTTTTGT TTGCAAGCAG CAGATTACGC GCAGAAAAAA
9301 AGGATCTCAA GAAGATCCTT TGATCTTTTC TACGGGGTCT GACGCTCAGT
9351 GGAACGAAAA CTCACGTTAA GGGATTTTGG TCATGAGATT ATCAAAAAGG
9401 ATCTTCACCT AGATCCTTTT AAATTAAAAA TGAAGTTTTA AATCAATCTA
9451 AAGTATATAT GAGTAAACTT GGTCTGACAG TTACCAATGC TTAATCAGTG
9501 AGGCACCTAT CTCAGCGATC TGTCTATTTC GTTCATCCAT AGTTGCCTGA
9551 CTCCCCGTCG TGTAGATAAC TACGATACGG GAGGGCTTAC CATCTGGCCC
9601 CAGTGCTGCA ATGATACCGC GAGACCCACG CTCACCGGCT CCAGATTTAT
9651 CAGCAATAAA CCAGCCAGCC GGAAGGGCCG AGCGCAGAAG TGGTCCTGCA

9701 ACTTTATCCG CCTCCATCCA GTCTATTAAT TGTTGCCGGG AAGCTAGAGT
9751 AAGTAGTTCG CCAGTTAATA GTTTGCGCAA CGTTGTTGGC ATTGCTACAG
9801 GCATCGTGGT GTCACGCTCG TCGTTTGGTA TGGCTTCATT CAGCTCCGGT
9851 TCCCAACGAT CAAGGCGAGT TACATGATCC CCCATGTTGT GCAAAAAAGC
9901 GGTTAGCTCC TTCGGTCCTC CGATCGTTGT CAGAAGTAAG TTGGCCGCAG
9951 TGTTATCACT CATGGTTATG GCAGCACTGC ATAATTCTCT TACTGTCATG
10001 CCATCCGTAA GATGCTTTTC TGTGACTGGT GAGTACTCAA CCAAGTCATT
10051 CTGAGAATAC CGCGCCCGGC GACCGAGTTG CTCTTGCCCG GCGTCAATAC
10101 GGGATAATAG TGTATGACAT AGCAGAACTT TAAAAGTGCT CATCATTGGA
10151 AAACGTTCTT CGGGGCGAAA ACTCTCAAGG ATCTTACCGC TGTTGAGATC
10201 CAGTTCGATG TAACCCACTC GTGCACCCAA CTGATCTTCA GCATCTTTTA
10251 CTTTCACCAG CGTTTCTGGG TGAGCAAAAA CAGGAAGGCA AAATGCCGCA
10301 AAAAAGGGAA TAAGGGCGAC ACGGAAATGT TGAATACTCA TACTCTTCCT
10351 TTTTCAATAT TATTGAAGCA TTTATCAGGG TTATTGTCTC ATGAGCGGAT
10401 ACATATTTGA ATGTATTTAG AAAAATAAAC AAATAGGGGT TCCGCGCACA
10451 TTTCCCCGAA AAGTGCCACC TGTATGCGGT GTGAAATACC GCACAGATGC
10501 GTAAGGAGAA AATACCGCAT CAGGCGACGC GCCCTGTAGC GGCGCATTAA
10551 GCGCGGCGGG TGTGGTGGTT ACGCGCAGCG TGACCGCTAC ACTTGCCAGC
10601 GCCCTAGCGC CCGCTCCTTT CGCTTTCTTC CCTTCCTTTC TCGCCACGTT
10651 CGCCGGCTTT CCCCGTCAAG CTCTAAATCG GGGGCTCCCT TTAGGGTTCC
10701 GATTTAGAGC TTTACGGCAC CTCGACCGCA AAAAACTTGA TTTGGGTGAT
10751 GGTTCACGTA GTGGGCCATC GCCCTGATAG ACGGTTTTTC GCCCTTTGAC
10801 GTTGGAGTCC ACGTTCTTTA ATAGTGGACT CTTGTTCCAA ACTGGAACAA
10851 CACTCAACCC TATCTCGGTC TATTCTTTTG ATTTATAAGG GATTTTGCCG
10901 ATTTCGGCCT ATTGGTTAAA AAATGAGCTG ATTTAACAAA TATTTAACGC
10951 GAATTTTAAC AAAATATTAA CGTTTACAAT TTCCATTCGC CATTCAGGCT
11001 GCGCAACTGT TGGGAAGGGC GATCGGTGCG GGCCTCTTCG CTATTACGCC
11051 AGCTGGCGAA AGGGGGATGT GCTGCAAGGC GATTAAGTTG GGTAACGCCA
11101 GGGTTTTCCC AGTCACGACG TTGTAAAACG ACGGCCAGTG AATTGTAATA
11151 CGACTCACTA TA

## Claims

1. A cell lines that express human mutated tissue-type plasminogen activator (TNK-TPA), its Collection NO. is CCTCC C200006.

2. A construction strategy of the cell lines said in claim 1, includes the following steps:
(1) Using a DNA sequence from the short arms of human D,G group chromosomes, or its homologous sequence as leading sequence for gene targeting to construct a human source gene vector, in which no important physiological function gene is found.
(2)The target gene TNK-TPA is ligated into the human source vector above with routine method, and the recombinant of the vector and TNK-TPA is obtained.
(3)TNK-TPA gene is targeted to nucleolus organizing region of human D,G group chromosomes in host cell HT1080 by the above recombinant and the cell line attained by screening.

3. The construction strategy of the cell lines according to claim 2 wherein the vector has the DNA sequence as shown in the SEQ No. 1.

4. The method of preparing mutated human tissue-type plasminogen activator is that the cell lines mentioned in Claim 1 are used as engineering cell line to express and manufacture TNK-TPA protein.
